(19)

Europäisches Patentamt
European Patent Office
Office européen des brevets

(11) **EP 3 951 391 A1**

(12) **EUROPEAN PATENT APPLICATION**
published in accordance with Art. 153(4) EPC

(43) Date of publication:
**09.02.2022 Bulletin 2022/06**

(21) Application number: **20777515.6**

(22) Date of filing: **25.03.2020**

(51) International Patent Classification (IPC):
*G01N 33/543* (2006.01)     *G01N 33/553* (2006.01)

(52) Cooperative Patent Classification (CPC):
**G01N 33/543; G01N 33/553**

(86) International application number:
**PCT/JP2020/013207**

(87) International publication number:
**WO 2020/196581 (01.10.2020 Gazette 2020/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.03.2019   JP 2019058402**

(71) Applicants:
• **Sekisui Medical Co., Ltd.**
  **Tokyo 103-0027 (JP)**
• **Sekisui Chemical Co., Ltd.**
  **Osaka 530-8565 (JP)**

(72) Inventors:
• **YAJI, Maasa**
  **Tokyo 103-0027 (JP)**

• **INABA, Yuya**
  **Tokyo 103-0027 (JP)**
• **KITAHARA, Shinichiro**
  **Tokyo 103-0027 (JP)**
• **YAMAMOTO, Mitsuaki**
  **Tokyo 103-0027 (JP)**
• **WAKIYA, Takeshi**
  **Mishima-gun, Osaka 618-0021 (JP)**
• **YAMAGAMI, Mai**
  **Mishima-gun, Osaka 618-0021 (JP)**
• **OBINATA, Shuhei**
  **Mishima-gun, Osaka 618-0021 (JP)**

(74) Representative: **Hoffmann Eitle**
**Patent- und Rechtsanwälte PartmbB**
**Arabellastraße 30**
**81925 München (DE)**

(54) **MAGNETIC RESPONSIVE PARTICLE AND IMMUNOASSAY METHOD USING SAME, REAGENT FOR IMMUNOASSAY**

(57) Disclosed is a sensitized magnetic responsive particle including: a magnetic responsive particle having a core particle and at least one magnetic layer that is disposed on the core particle and includes microparticles of a magnetic metal and/or an oxide thereof; and a substance that is supported on the magnetic responsive particle and interacts specifically with an analyte, wherein the coefficient of variation in the weight-average particle size of the magnetic responsive particles is 15% or less. The magnetic responsive particle provided has excellent magnetic separability.

EP 3 951 391 A1

**Description**

Technical Field

The present invention relates to a magnetic responsive particle used in a reagent for an immunoassay and an immunoassay method and an immunoassay reagent using the particle.

Background Art

As a procedure for measuring and/or purifying a protein of interest from a biological substance-containing sample, an assay has been known, including: immobilizing, on a solid support, a substance interacting specifically with an analyte; causing the substance to bind to the analyte in a biological sample; washing away unbound substances other than the analyte; and measuring the quantity of the analyte bound to the solid support.

Because the unbound substances are easy to be separated and collected when removed, a magnetic responsive particle is used as the solid support. For instance, Patent Literature 1 discloses, as such a particle, a clinical test reagent particle having a magnetic layer formed on the surface of a core particle and a polymer layer formed thereon. With regard to the particle disclosed, however, only particles with a broad range of particle size are obtained.

Patent Literature 2 discloses a particle enabling highly sensitive immunoassay, the particle having a markedly increased magnetic material content because a nonmagnetic metal oxide is subjected to polycondensation in the co-presence of magnetic material. However, the production method disclosed merely gave particles with a broad particle size distribution. Thus there were a problem of poor assay reproducibility when used as a clinical reagent and a concern about the lack of an accurate measurement because the excess magnetic material content affects an immunological reaction. Besides, the particle disclosed includes a superparamagnetic metal oxide particle in a nonmagnetic oxide. Thus it has a high specific gravity and an increased precipitability, causing a problem in that the particle is difficult to handle when used as an immunoassay reagent.

Citation List

Patent Literature

Patent Literature 1: Japanese Patent Laid-Open No. 2004-205481
Patent Literature 2: Japanese Patent Laid-Open No. 2013-019889

Summary of Invention

Technical Problem

The purpose of the present invention is to provide an immunoassay reagent having excellent magnetic separability and high sensitivity potential, and a magnetic responsive particle used therefor.

Solution to Problem

The present inventors have conducted intensive research to solve the above-described problems, and have found that when the magnetic material content of each magnetic responsive particle is made constant, a magnetic responsive particle having favorable magnetic separability and capable of realizing a highly sensitive immunoassay reagent can be obtained. Specifically, the invention relates to the following items.

[1] A sensitized magnetic responsive particle including: a magnetic responsive particle having a core particle and at least one magnetic layer disposed on the core particle, the magnetic layer including microparticles of a magnetic metal and/or an oxide thereof; and a substance that specifically interacts with an analyte, the substance being supported on the magnetic responsive particle, wherein a coefficient of variation in a weight-average particle size of the magnetic responsive particles is 15% or less.

[2] The sensitized magnetic responsive particle according to [1], wherein the coefficient of variation in a volume-average particle size of the magnetic responsive particles is 20% or less.

[3] A sensitized magnetic responsive particle including: a magnetic responsive particle having a core particle and at least one magnetic layer disposed on the core particle, the magnetic layer including microparticles of a magnetic metal and/or an oxide thereof; and a substance that specifically interacts with an analyte, the substance being supported on the magnetic responsive particle, wherein a coefficient of variation in a weight-average particle size of the sensitized magnetic responsive particles is 15% or less.

[4] The sensitized magnetic responsive particle according to [3], wherein the coefficient of variation in a volume-average particle size of the sensitized magnetic responsive particles is 20% or less.

[5] The sensitized magnetic responsive particle according to any one of [1] to [4], further including a nonmagnetic layer including a nonmagnetic metal oxide and/or an organic metal compound between the magnetic layer and the substance interacting specifically with the analyte.

[6] The sensitized magnetic responsive particle according to any one of [1] to [5], wherein the substance interacting specifically with the analyte is chemically bonded onto the magnetic layer through a one-step or multi-step reaction.

[7] The sensitized magnetic responsive particle according to any one of [1] to [6], wherein the substance interacting specifically with the analyte is bonded onto the nonmagnetic layer via one or multiple chemical bonds.

[8] A heterogeneous immunoassay method using the sensitized magnetic responsive particle according to any one of [1] to [7].

[9] An immunoassay reagent including the sensitized magnetic responsive particle according to any one of [1] to [7].

Advantageous Effects of Invention

The invention can provide a magnetic responsive particle containing magnetic material uniformly, having excellent magnetic separability, and exerting a remarkably less analyte-binding inhibition on the surface, and an immunoassay reagent that uses the particle to deliver high performance.

[Description of Embodiments]

Hereinbelow, the invention will be described with reference to embodiments. However, the invention is not limited to the following embodiments.

1. Magnetic Responsive Particle and Production Method Thereof

Hereinafter, each element, etc. will be described in detail.

1.1 Core Particle

A magnetic responsive particle of the invention has at least one magnetic layer that is disposed on a particle to be a core (core particle) and includes microparticles of a magnetic metal and/or an oxide thereof.

The core particle of the invention may be made of inorganic or organic material and is not particularly limited. But a resin particle constituted by a resin is preferred in the case of use as an immunoassay reagent because a smaller specific gravity gives better dispersibility. The resin particle is basically a nonmagnetic material, and for instance, it is possible to use organic matter such as a polymer.

Examples of the material constituting the above resin particles include, but are not particularly limited to, polyolefins such as polyethylene, polypropylene, polystyrene, polyvinyl chloride, polyvinylidene chloride, polytetrafluoroethylene, polyisobutylene, polybutadiene; acrylic resins such as polymethylmethacrylate, polymethylacrylate; acrylate/divinylbenzene copolymer resins; polyalkylene terephthalate; polysulfone; polycarbonate; polyamide; phenol formaldehyde resins; melamine formaldehyde resins; benzoguanamine formaldehyde resins; and urea formaldehyde resins. These materials constituting the resin microparticles may be used singly, or two or more types thereof may be used in combination.

The core particle in the invention has an average particle size of preferably from 0.5 to 10 $\mu$m, more preferably from 1 to 5 $\mu$m, and most preferably from 2.5 to 4 $\mu$m. If the average particle size of the core particles is less than 0.5 $\mu$m, the area where magnetic material can be attached to each particle is small. This may cause insufficient magnetic separability. In addition, if the average particle size of the core particles exceed 10 $\mu$m, the surface area as a reaction site may be small when the core particle is utilized as a carrier for biochemical use after attached to magnetic material.

The magnetic separability is an indicator that indicates a response of magnetic responsive particle to a magnet. The magnetic separability may be evaluated by applying a magnet to an aqueous dispersion of magnetic responsive particles and calculating an attenuation of absorbance measured over time using, for instance, a spectrophotometer (U-3900H, manufactured by Hitachi, Ltd.). The larger the attenuation is, the better the response to a magnet is. When used as a reagent for an immunoassay, the magnetic responsive particles can be said to separate an analyte more efficiently in a short time.

In addition, the core particle has a coefficient of variation (CV value) in the volume-average particle size of 20% or less, preferably 15% or less, and more preferably 10% or less. Use of, as a core, a particle with a large CV value causes a variation in the surface area per particle. This may give a variation in the magnetic material coating amount when a magnetic layer is formed. The coating amount variation is undesirable because it leads to a variation in magnetic separability and thus the assay reproducibility may be deteriorated in the case of use as an immunoassay reagent. To control the particle size, it is possible to apply any methods of controlling a particle size: a method in which the particle-size controlling process is performed during a step of manufacturing core particles; or a method in which the particle-size controlling process is performed by classification after manufacturing core particles. In addition, these methods may be used in combination.

The average particle size in the invention may be determined by observing particles under, for instance, a scanning electron microscope ("S-4800", manufactured by Hitachi High-Technologies Corporation) and calculating the mean of the maximum diameter of respective 50 particles selected randomly in an image observed.

The volume-average particle size in the invention is a volume-average particle size obtained by measurement with, for instance, a laser diffraction and scattering particle size distribution analyzer ("LS 13 320", manufactured by Beckman Coulter Inc.).

The above core particle may be provided with a reactive functional group on the surface of the particle. This may be used, for instance, as a binding site at the time of coating with magnetic material.

The above core particle used may be a particle where liquid material or solid material fine powder is absorbed or adsorbed. This can produce a magnetic material-coated particle including, inside and/or on the surface, the above liquid material or solid material. Note that the above material absorption/adsorption means absorption/adsorption or attachment, etc. through/on the particle surface and the pore interior. This absorption and adsorption can be implemented by a conventionally known procedure such as impregnation.

### 1.2 Magnetic Material

As a magnetic metal and/or a magnetic metal oxide used for coating of the core particle surface in the invention, one type may be used singly or two or more types may be used in combination. In addition, the metal and/or metal oxide may be provided with a reactive functional group on the surface of the particle. This may be used, for instance, as a binding site when the core particle is coated.

From the viewpoint of magnetic separability, the magnetic metal and/or the magnetic metal oxide preferably includes at least one selected from any of groups 8 to 10 metals in periods 4 to 6 of the periodic table or lanthanoids. Alternatively, preferred is an iron oxide-based substance. Specific examples include a ferrite represented by $MFe_2O_4$ (where M = Co, Ni, Mn, Zn, Mg, Cu, $Li_{0.5}Fe_{0.5}$, etc.), a magnetite represented by $Fe_3O_4$, or $\gamma Fe_2O_3$. Most particularly preferred is $Fe_3O_4$ or $\gamma Fe_2O_3$ as a magnetic material having strong saturation magnetization and less residual magnetization.

### 1.3 Magnetic Layer

A magnetic metal-coated particle (hereinafter, referred to as a "magnetic responsive particle") in the invention has a magnetic layer formed by adsorbing, on the core particle surface, a magnetic metal and/or metal oxide. Here, the metal and/or metal oxide may be coated by physical adsorption or via a chemical bond(s) on the surface of the resin particle. The physical adsorption of the metal and/or metal oxide in the invention refers to adsorption/bonding without any chemical reaction. Examples include melt bonding or adsorption, fusion bonding or adsorption, hydrogen bonding, van der Waals bonding, electrostatic interaction, or heterogeneous aggregation. The coating via a chemical bond(s) means that a functional group provided on the surface of the resin particle is bonded through a chemical reaction to a functional group provided on the metal and/or the metal oxide, so that the surface of the core particle supports the metal and/or metal oxide on the surface of the core particle surface. Among these coating manners, coating by the physical adsorption is more preferable because the preparation is convenient.

Using magnetic metals and/or metal oxides, the formation of a complex may be repeated multiple times on the same core particles to produce magnetic responsive particles. In each step, the metals and/or metal oxides used for the formation of a complex are not particularly limited and may be used alone or in combination of two or more. In addition, the method for adding the metals and/or metal oxides is not particularly limited, and any of a batch process, a divided process, or a continuous addition process may be allowed. Further, when the metals and/or metal oxides are used in combination of two or more at a given ratio, the order of addition is not particularly limited. All of them may be mixed and added, each of them may be added separately, or some of them may be mixed and others may be added separately, etc. They may be added in any given order and combination. The number of additions is not particularly limited, either.

Furthermore, a functional material other than the magnetic material may be added, if necessary, as a magnetic layer-forming material, at the time of complex formation. The type of such functional material is not particularly limited, and may be selected, if appropriate, from organic matter or inorganic matter depending on the purpose of the formation of a complex. The kind is not limited to only a single type, and two or more types may be used in combination. The purpose herein refers to, for instance, adding a function(s) other than imparting magnetic separability, such as imparting electrical properties, coloring, or the like.

The formation of a complex may be repeated multiple times on the same core particles using magnetic material and/or functional materials. In each step, the magnetic materials and/or the functional materials used for the formation of a complex are not particularly limited. Only the magnetic material may be used, or only the functional material may be used. Alternatively, both the magnetic material and the functional material may be used. In addition, one type from any of the magnetic material or the functional material may be used singly, or two or more types may be used in combination. Further, each process of adding the material is not particularly limited. Any of a batch process, a divided process, or a continuous addition process may be allowed. When two or more thereof are used in combination at a given ratio, the order of addition is not particularly limited. All of them may be mixed and added, each may be added separately, or some of them may be mixed and others may be added separately, etc. They may be added in any given order and combination. The number of additions is also not particularly limited.

When the complex formation is repeated multiple times on the same core particles using magnetic metals and/or metal oxides, a nonmagnetic layer may be formed on the magnetic layer, followed by further formation of a magnetic layer. The magnetic layer and the nonmagnetic layer may be alternately formed to form a plurality of layers.

The magnetic material content of magnetic responsive particles is preferably from 10 wt% to 50 wt%. If the magnetic material content is 50 wt% or more, the specific gravity of the magnetic responsive particles as a final product is large, and the precipitability is increased. This causes a concern about poor particle dispersibility. In addition, as the magnetic material content becomes higher, the coefficient of variation (CV value) in the weight-average particle size increases, which may cause an adverse effect on reproducibility in the case of use as an immunoassay reagent. If the content is less than 10 wt%, sufficient magnetic separability cannot be achieved, and thus, the separation and collection become difficult in the case of use as an immunoassay reagent.

The magnetic responsive particle dispersibility may be evaluated by a dispersion rate. The dispersion rate can be calculated by a change rate of absorbance from absorbance before magnetic collection of the magnetic responsive particles and absorbance after magnetic collection and dispersion. The dispersion rate is 85% or more, preferably 90% or more, and still more preferably 95% or more. If the dispersion rate is less than 85%, magnetic particles after magnetic separation are not sufficiently re-dispersed, which may cause a decrease in assay precision, assay sensitivity, and assay reproducibility.

The coefficient of variation (CV value) in the weight-average particle size of the magnetic responsive particles is preferably 15% or less and more preferably 10% or less. The CV value for the weight-average particle size indicates how the particle size varies and how the density varies. A lower CV value indicates a uniform particle size and a uniform density. A higher CV value indicates an ununiform particle size or density, or indicates that both are ununiform. The CV value for the weight-average particle size in the invention is a value obtained with, for instance, a disc centrifugation-type particle size distribution analyzer ("DC24000UHR", manufactured by CPS Instruments, Inc.). The CV value for the weight-average particle size of the particles in the invention is as low as 10% or less, the particles in the invention each have a similar magnetic material content (amount). Thus, the magnetic separability is better than those of conventional magnetic responsive particles.

The magnetic responsive particles have an average particle size of preferably from 0.5 to 10 $\mu$m, more preferably from 1 to 8 $\mu$m, and still more preferably from 2 to 5 $\mu$m. The average particle size in the invention is a value obtained with, for instance, a scanning electron microscope ("S-4800", manufactured by Hitachi High-Technologies Corporation).

The CV value for the volume-average particle size of the magnetic responsive particles is 20% or less, preferably 15% or less, and more preferably 10% or less. The CV value for the volume-average particle size reflects a variation in the particle size. This indicates that if the value is low, the particle size is uniform, and if the value is high, the particle size is ununiform.

The magnetic separability of the magnetic responsive particles is preferably 40% or more, more preferably 50% or more, and most preferably 60% or more.

Note that the magnetic layer composed of a metal and/or metal oxide has a thickness of from about 10 to 200 nm; the below-described nonmagnetic layer composed of a metal oxide and/or organic metal compound has a thickness of from about 10 to 500 nm; and the finally obtained magnetic responsive particles have an average particle size of from about 0.5 to 10 $\mu$m.

1.4 Nonmagnetic Layer

The magnetic responsive particles in the invention may have a nonmagnetic metal oxide layer and/or a nonmagnetic organic metal compound layer on the magnetic layer surface. This nonmagnetic layer is formed to coat the magnetic layer and/or impart own functionalities to the particle.

With regard to usage as a carrier for biochemical use, such as an immunoassay reagent, properties of the surface of the nonmagnetic layer may be selected depending on the purpose. The below-described procedure may be used for the formation of the nonmagnetic layer to strongly prevent impurities from eluting from the particles, magnetic material itself from eluting, or impurities from eluting from the magnetic layer. This can realize a more preferred state, in particular, as carrier particles for an immunoassay reagent.

The nonmagnetic layer may be formed by adding, in the presence of a particle, a nonmagnetic metal oxide and/or a nonmagnetic organic metal compound as a main raw material and, optionally, other auxiliary materials and reacting them in a liquid phase. The nonmagnetic metal oxide and/or the nonmagnetic organic metal compound used at this time preferably have a functional group that can react with a surface of the magnetic material. Use of the metal oxide and/or the organic metal compound that can directly react with a surface of the magnetic material enables the magnetic layer and the nonmagnetic layer to bind together strongly and keep them highly tightly attached, and thus can cause superior effects of preventing leakage of magnetic layer components and immobilizing the components. Meanwhile, in the case of using a radical polymerizable monomer represented by, for instance, a vinyl-based monomer, the monomer is not directly bonded to the magnetic material. Thus, attachment between the magnetic layer and the polymer layer is poor, and the magnetic layer components are insufficiently immobilized. As a result, leakage of the components and destabilization of the shape/magnetic separability may be caused.

A procedure for reacting the magnetic surface and the nonmagnetic layer is not particularly limited. Examples include covalent bonding or coordination bonding.

The following will describe main raw materials of the nonmagnetic layer. For convenience, just a single molecule compound is exemplified. However, a compound containing, in a molecule, one or more functional groups that can react with a surface of the magnetic material is acceptable. The compound may be a dimer to a multimer in which multiple single molecules are subjected to polycondensation. In addition, the number of contained functional groups that can react with the magnetic material is not particularly limited.

The nonmagnetic metal oxide and/or the nonmagnetic organic metal compound preferably contain at least one selected from Si, Ge, Ti, or Zr. As mentioned above, it is preferable to have a functional group(s) that can react with a surface of the magnetic layer. Specific examples include: silane compounds represented by alkoxysilane such as tetraethyl orthosilicate and its hydrolysis products; germanium compounds represented by alkoxygermanium such as germanium tetraethoxide and its hydrolysis products; titanium compounds represented by alkoxytitanium such as titanium tetraethoxide and its hydrolysis products; or zirconium compounds represented by alkoxyzirconium such as zirconium tetrabutoxide and its hydrolysis products. Here, in view of maintaining particle dispersibility, the specific gravity of the nonmagnetic layer should be as small as possible. Among the above examples, a silane compound is most preferable.

Further, in the metal oxide and/or the organic metal compound used as a main raw material for the nonmagnetic layer, a metal oxide and/or an organic metal compound having another functional moiety in addition to the functional group(s) that reacts with a surface of the magnetic layer may be used. In this case, the magnetic responsive particles may also be provided with another function derived from the metal oxide and/or the organic metal compound.

The metal oxide and/or the organic metal compound having another functional moiety will be specifically exemplified with reference to silane compounds. However, the metal oxide and/or the organic metal compound used are not limited to them.

Examples include vinyl-containing compounds such as vinyltrimethoxysilane, vinyltriethoxysilane, 7-octenyltrimethoxysilane; epoxy-containing compounds such as 2-(3,4-epoxycyclohexyl)ethyltrimethoxysilane, 3-glycidoxypropylmethyldimethoxysilane, 3-glycidoxypropyltrimethoxysilane, 3-glycidoxypropylmethyldiethoxysilane, 3-glycidoxypropyltriethoxysilane, 8-glycidoxyoctyltrimethoxysilane; styryl-containing compounds such as p-styryltrimethoxysilane; methacryl-containing compounds such as 3-methacryloxypropylmethyldimethoxysilane, 3-methacryloxypropyltrimethoxysilane, 3-methacryloxypropylmethyldiethoxysilane, 3-methacryloxypropyltriethoxysilane, 8-methacryloxyoctyltrimethoxysilane; acryl-containing compounds such as 3-acryloxypropyltrimethoxysilane; amino-containing compounds such as N-2-(aminoethyl)-3-aminopropylmethyldimethoxysilane, N-2-(aminoethyl)-3-aminopropyltrimethoxysilane, 3-aminopropyltrimethoxysilane, 3-aminopropyltriethoxysilane, 3-triethoxysilyl-N-(1,3-dimethylbutylidene)propylamine, N-phenyl-3-aminopropyltrimethoxysilane, N-(vinylbenzyl)-2-aminoethyl-3-aminopropyltrimethoxysilane hydrochloride, N-2-(aminoethyl)-8-aminooctyltrimethoxysilane; isocyanurate-containing compounds such as tris-(trimethoxysilylpropyl)isocyanurate; ureido-containing compounds such as 3-ureidopropyltrialkoxysilane; mercapto-containing compounds such as 3-mercaptopropylmethyldimethoxysilane, 3-mercaptopropyltrimethoxysilane; isocyanate-containing compounds such as 3-isocyanate propyltriethoxysilane; carboxylic acid anhydride-containing compounds such as 3-trimethoxysilylpropyl succinic acid anhydride; and carboxylic acid-containing compounds such as hydrolyzed 3-trimethoxysilylpropyl succinic acid anhydride).

When used as a carrier for biochemical use, preferred are epoxy-containing compounds, amino-containing compounds, mercapto-containing compounds, carboxylic acid anhydride-containing compounds, or carboxylic acid-containing compounds among them. Likewise, preferred examples include: those in which a double bond portion(s) of vinyl-containing compound/styryl-containing compound is oxidized to epoxidize; or those in which a vinyl-containing compound/styryl-containing compound is introduced onto a particle, and its double bond portion is then oxidized to give an epoxy group, which have increased reactivity with a bio-related material by converting a functional group(s) before or after introduction onto the particle.

The metal oxides and/or the organic metal compounds used as a main raw material for the nonmagnetic layer as described above may be a single type, or two or more types may be used in combination at a given ratio. In the case of using two or more types of the metal oxide and/or the organic metal compound are used, the combination of the two or more types of the metal oxide and/or the organic metal compound is any of a combination of two or more types of a metal oxide and/or organic metal compound having another functional moiety, a combination of two or more types of a metal oxide and/or an organic metal compound without another functional moiety, or a combination of a metal oxide and/or an organic metal compound having one or more other functional moieties and a metal oxide and/or an organic metal compound without one or more other functions.

The procedure for adding the metal oxide and/or the organic metal compound during the formation of the nonmagnetic layer is not particularly limited, and any of a batch process, a divided process, or a continuous addition process may be allowed. Further, in the case of using, at a given ratio, two or more types in combination at a given ratio, the order of addition is not particularly limited. All of them may be mixed and added, each may be added separately, or some of them may be mixed and others may be added separately, etc. They may be added in any given order and combination. The number of additions is also not particularly limited.

Coating of the nonmagnetic layer and coating of the magnetic layer may be repeated on the core particle, the surface of which has been coated with the magnetic layer. For instance, the core particle, the surface of which has been coated with the magnetic layer, is coated with a nonmagnetic layer. This surface layer may be further coated with a magnetic layer and a nonmagnetic layer in this order. At this time, as long as at least one magnetic layer is included, and the outermost surface layer is a nonmagnetic layer, the number of coatings (the number of layers) as well as the number and types of magnetic layers/nonmagnetic layers (inside) in the whole coating layers on the core particle are not limited. Note that the case of including two or more magnetic layers, the magnetic material content of the higher final particles is improved compared with the particles including only one magnetic layer, so that the magnetic response can be made higher.

At the time of the formation of the nonmagnetic layer, an auxiliary material, in addition to the metal oxide and/or the organic metal compound as a main raw material, may be optionally used. The auxiliary material is not particularly limited. For instance, in the case of reacting the magnetic material surface with the metal oxide and/or the organic metal compound in the invention, an acid or base may be typically added to proceed with the reaction in many cases.

The coefficient of variation (CV value) in the weight-average particle size of the magnetic responsive particle having a nonmagnetic layer formed on the surface of the magnetic layer is preferably 15% or less and more preferably 10% or less. In addition, the average particle size is preferably from 0.5 to 10 $\mu$m, more preferably from 1 to 8 $\mu$m, and still more preferably from 2 to 5 $\mu$m. The CV value for the volume-average particle size is 20% or less, preferably 15% or less, and more preferably 10% or less.

1.5 Substance for Support and How to Support Bio-Related Material

<Analyte>

An analyte in the invention means a substance to be measured/captured. For instance, this substance is present in the body or in a biological sample such as blood (whole blood), erythrocytes, serum, plasma, urine, saliva, or sputum. Examples include: inflammation-related markers such as a diseased tissue, a diseased cell, CRP (C-reactive protein), IgA, IgG, IgM; coagulation and fibrinolysis markers such as fibrin degradation products such as D-dimers), soluble fibrin, TAT (a thrombin-antithrombin complex), PIC (a plasmin-plasmin inhibitor complex); circulation-related markers such as oxidized LDL, BNP (brain natriuretic peptide), H-FABP (cardiac fatty acid binding protein), cardiac troponin I (cTnI); metabolism-related markers such as adiponectin; tumor markers such as CEA (Cancer Embryonic Antigen), AFP ($\alpha$-Fetoprotein), PIVKA-II, CA19-9, CA125, PSA (Prostate Specific Antigen); infection-related markers such as HBV (hepatitis B virus), HCV (hepatitis C virus), *Chlamydia trachomatis, Neisseria gonorrhoeae;* respiratory-related markers such as KL-6; allergen-specific IgE (immunoglobulin E); hormones; and drugs.

<Interacting Substance>

Examples of the substance specifically interacting with an analyte in the invention include proteins, peptides, amino acids, lipids, carbohydrates, DNA, RNA, receptors, haptens, biotin, and avidin. How high or low the molecular weight is or whether the interacting substance is derived from a naturally occurring or synthesized one is not particularly limited. Examples include an antibody(s) or an antigen(s) that can be used in an immunoassay utilizing an immunological reaction. Meanwhile, the term "interacting" means a reaction or binding.

Main usage of the above magnetic responsive particles is a carrier for biochemical use, such as an immunoassay reagent. A magnetic responsive particle as a carrier for biochemical use (hereinafter, referred to as a "sensitized magnetic responsive particle") may be produced by using the particle as a carrier and immobilizing an analyte, an analyte analogue, or a substance specifically interacting with an analyte (hereinafter, sometimes generally referred to in short as a "substance for support").

The procedure for immobilizing a substance for support on the magnetic responsive particle to produce a sensitized magnetic responsive particle of the invention is not particularly limited. Conventionally known physical and/or chemical bonding may be used for the immobilization. When immobilized via a chemical bond, by forming a nonmagnetic layer by using a metal oxide and/or an organic metal compound having a bio-related material-binding functional group as exemplified in paragraph 0042, the functional group present on the surface of the magnetic responsive particle can be immobilized as a scaffold for binding of the substance for support.

The above technique is preferably used to provide a surface of the magnetic responsive particle with an epoxy group, an amino group, a mercapto group, a carboxylic acid group, or a carboxylic acid anhydride structure and support a substance for support via each structure on a surface of the particle.

In addition, a bio-related material that can specifically bind to the above substance for support may be supported on the magnetic responsive particle, and the substance for support may be bound via the bio-related material to the magnetic responsive particle in a reaction system. Examples of the bio-related material that can be used for the purpose include avidin and streptavidin. The magnetic responsive particle on which a bio-related material is supported so as to mediate the binding to a substance for support may also be regarded as a sensitized magnetic responsive particle.

The resulting sensitized magnetic responsive particle may be optionally coated (blocked) with each polymer compound or protein (e.g., bovine serum albumin), and may be dispersed in a suitable buffer and then used as a sensitized magnetic particle dispersion. The sensitized particle dispersion may be used as a reagent for an immunoassay. Further, a diluent (buffer) and/or a standard substance, etc., utilized for the assay may be used in combination to provide an assay reagent kit.

The coefficient of variation (CV value) in the weight-average particle size of the sensitized magnetic responsive particles is preferably 15% or less and more preferably 10% or less. In addition, the average particle size is preferably from 0.5 to 10 $\mu$m, more preferably from 1 to 8 $\mu$m, and most preferably from 2 to 5 $\mu$m. The CV value for the volume-average particle size is 20% or less, preferably 15% or less, and more preferably 10% or less.

The reagent and the diluent for an immunoassay may contain various sensitizers in order to increase the assay sensitivity and facilitate a specific reaction between an analyte and the substance for support. In addition, the reagent and the diluent for an immunoassay may contain, for instance, various polymer compounds/proteins and their degradation products, amino acids, and/or surfactants in order to suppress a nonspecific reaction caused by a substance(s) other than an assay target substance present in an assay sample and to improve stability of the assay reagent.

The method for assaying an assay target substance using an immunoassay reagent of the invention is not particularly limited as long as the magnetic responsive particle in the invention is used. For instance, a sandwich assay, a competition assay, or the like, which is usually performed in the art, may be carried out in accordance with the description of a literature (e.g., "Enzymatic Immunoassay, 2nd edition", edited by Eiji Ishikawa, et al., Igaku-Shoin Ltd., 1982).

The analyte assay includes the steps of: bringing a sample, a sensitized magnetic responsive particle, a labeled analyte-binding substance, a labeled assay target substance or its analogue, etc., into contact; and implementing B/F separation (separating a bound labeled antibody from a free labeled antibody). In the former step, the magnetic particles may be dispersed by regular treatment such as stirring or mixing. The latter step is carried out by, for instance, utilizing magnetism of magnetic particles to collect the magnetic particles by using a magnet or the like applied from the outside of a reaction vessel, etc.; discharging the reaction solution; adding a washing solution; removing the magnet; and mixing, dispersing, and then washing the magnetic particles. The above operations may be repeated once to three times. The washing solution is not particularly limited as long as the solution is routinely used in the art.

The results of measuring an analyte may be calculated from a value obtained by labeling an analyte or its analogue, etc., with a label substance and measuring the quantity or activity. A routine protocol may be used as the procedure for measuring a label substance or its activity, which is not particularly limited. Specific examples include radioimmunoassay (RIA), enzyme immunoassay (EIA), fluorescence immunoassay (FIA), electrochemiluminescence immunoassay (ECLIA), chemiluminescence immunoassay (CLIA and CLEIA), absorbance measurement, or surface plasmon resonance. An optical instrument used at the time of measurement is not particularly limited. Any of biochemical automatic analyzers widely used in clinical tests as a representative example may be used.

In the case where the magnetic responsive particle is utilized as a carrier for biochemical use, such an immunoassay reagent, as described above, it is critical to have increased magnetic separability. Use of particles with excellent magnetic separability allows for superior washing/purification efficiency and a decreased particle loss. This can elicit high performance of, in particular, an immunoassay reagent. The above magnetic responsive particle of the invention can realize high magnetic separability, and is thus fit for a carrier for biochemical use, such as an immunoassay reagent.

Examples

2. Examples

Hereinafter, the invention will be described in more detail with reference to Examples. However, the invention is not limited by them.

The following protocols were used to measure the average particle size and its CV value, the CV value for the average density, the magnetic separability, and the magnetic material content of particles obtained in each of Examples or Comparative Examples.

2.1 Protocols for Measuring Physical Properties

2.1.1 Average Particle Size

The average particle size was determined by observing particles under a scanning electron microscope ("S-4800", manufactured by Hitachi High-Technologies Corporation) and calculating the mean of the maximum diameter of respective 50 particles selected randomly in an image observed.

2.1.2 Measurement of CV Value in Volume-Average Particle Size

The CV value for the volume-average particle size was calculated by measuring a volume-average particle size distribution by using a laser diffraction and scattering particle size distribution analyzer ("LS 13 320", manufactured by Beckman Coulter Inc.).

2.1.3 Measurement of CV Value for Weight-Average Particle Size

The CV value for the weight-average particle size was calculated by measuring a weight-average particle size distribution by using a disc centrifugation-type particle size distribution analyzer ("DC24000UHR", manufactured by CPS Instruments, Inc.). Specifically, a solution having a density gradient obtained by mixing 8% and 24% sucrose solutions was centrifuged at 5000 rpm while a particle aqueous dispersion (0.1 mL), the absorbance of which had been adjusted to 1.0, was placed thereon for measurement.

2.1.4 Evaluation of Magnetic Separability

A spectrophotometer (U-3900H, manufactured by Hitachi, Ltd.) was used to measure absorbance at 550 nm for evaluation. While a magnet (2800 G, W10 mm × D10 mm × H1 mm) was attached onto the bottom of a quartz cell set in the spectrophotometer, a particle aqueous dispersion (1.3 mL), the absorbance of which had been adjusted to 1.0, was placed thereinto. At 5 sec or 125 sec after the sample placement, absorbance was read. An absorbance attenuation during this 120 sec was calculated to give an indicator for magnetic separability.

2.1.5 Measurement of Magnetic Material Content

The magnetic material content of the magnetic responsive particle can be calculated from a residue obtained by decomposing a resin portion by heating the particle in the air up to 1000°C. Specifically, the dry weight (A) of the magnetic responsive particle was accurately weighed; a simultaneous thermogravimetric analyzer (TG-DTA6300, manufactured by Hitachi High-Tech Science Corporation) was used to raise a temperature from 35°C to 1000°C at a programming rate of 5°C/min; maintaining the temperature at 1000°C for 5 min; then measuring the weight (B) of the resulting residue; and calculating the proportion of B to A as a percentage to give a magnetic material content.

2.1.6 Evaluation of Dispersibility

The sample solution used was a magnetic particle aqueous dispersion in which the absorbance at a wavelength of 550 nm had been adjusted to 1.0. Here, 1.3 mL of each sample solution was dispensed into a quartz cell set in a spectrophotometer ("U-3900H", manufactured by Hitachi, Ltd.), and absorbance at a wavelength of 550 nm was read. Next, a magnet (28000 G, W40 mm × D40 mm × H10 mm) was used to magnetically collect the particles in the solution until the absorbance of supernatant became 0. Then, the solution was vortexed at 2000 rpm for 5 sec to disperse the magnetic particles, and absorbance at a wavelength of 550 nm was measured. The absorbance before magnetic collection and the absorbance after magnetic collection and dispersion were used to calculate, using the following equation, a change in the absorbance to give a dispersion rate.

$$\text{Dispersion rate (\%)} = [(\text{Absorbance after magnetic collection and dispersion}) / (\text{Absorbance before magnetic collection})] \times 100.$$

2.2.1 Example 1-1

First, 2.0 g of Micropearl EX-003 (with a particle size of 3.01 $\mu$m and a CV of 3.1%, manufactured by SEKISUI CHEMICAL CO., LTD.) as resin particles was ultrasonically dispersed into 40.0 g of ion-exchanged water to obtain a core particle dispersion.

Subsequently, 4.0 mL of magnetic fluid EMG707 (manufactured by Ferrotec Corporation) was added while stirring under ultrasonic irradiation to perform ultrasonic dispersing treatment for additional 30 min. The resulting dispersion was filtered and washed with ion-exchanged water, and then excessive magnetic fluid was removed to yield magnetic responsive particles [1].

2.2.2 Example 1-2

First, 1.0 g of the magnetic responsive particles [1] obtained in Example 1-1 were ultrasonically dispersed into 400 g of ethanol. Next, 10 mL of 28% aqueous ammonia solution (manufactured by NACALAI TESQUE, INC.), 1.0 g of tetraethyl orthosilicate, and 3.0 g of 8-glycidoxyoctyltrimethoxysilane were added thereto and ultrasonically dispersed for 3 h. After the resulting dispersion was filtered, the dispersion into ion-exchanged water and centrifugation were repeated three times to give magnetic responsive particles EP [1] having an epoxy group on their surface.

The above magnetic responsive particles EP [1] were ultrasonically dispersed at 3.0 wt% in PBS, and 0.5 mL thereof was transferred to a test tube. After the magnetic responsive particles EP [1] were collected using a magnet onto a wall surface of the test tube, the dispersion medium was removed. Then, 0.5 mL of a PBS solution containing an anti-KL-6 antibody (0.75 mg/mL) was added. The mixture was stirred overnight at 25°C for sensitization to prepare anti-KL-6 antibody-sensitized magnetic responsive particles [1]. After that, 1.5 mL of 1.0 wt% BSA solution was added, and the mixture was stirred at 25°C for 4 h. The sensitized magnetic responsive particles were collected using a magnet onto a wall surface of the test tube. Thereafter, the dispersion medium was removed, and 1.5 mL of 1.0 wt% BSA solution was newly added and dispersed. This operation was repeated three times to prepare an anti-KL-6 antibody-sensitized magnetic responsive particle [1] dispersion.

2.2.3 Example 1-3

Streptavidin was dissolved into a 0.1 M boric acid aqueous solution to prepare a 0.1 $\mu$g/mL streptavidin solution.

The magnetic responsive particles EP [1] obtained in Example 1-2 were ultrasonically dispersed at 3.0 wt% in 0.1 M boric acid aqueous solution, and 0.5 mL thereof was transferred to a test tube. After the magnetic responsive particles EP [1] were collected using a magnet onto a wall surface of the test tube, the dispersion medium was removed. Next, 0.5 mL of the above streptavidin solution was added, and the mixture was stirred at 37°C for 18 h to yield streptavidin-sensitized magnetic responsive particles [1]. Then, 0.5 mL of 1.0 wt% BSA solution was added, and the mixture was stirred at a reaction temperature of 37°C for 4 h. The sensitized magnetic responsive particles were collected using a magnet onto a wall surface of the test tube. Thereafter, the dispersion medium was removed, and 1.5 mL of 1.0 wt% BSA solution was newly added and dispersed. This operation was repeated three times to prepare a streptavidin-sensitized magnetic responsive particle [1] dispersion.

2.2.4 Example 2-1

First, 2.0 g of Micropearl SP-203 (with a particle size of 3.02 $\mu$m and a CV of 4.9%, manufactured by SEKISUI CHEMICAL CO., LTD.) as resin particles was ultrasonically dispersed into 40.0 g of ion-exchanged water to prepare a core particle dispersion.

Subsequently, 4.0 mL of magnetic fluid EMG707 (manufactured by Ferrotec Corporation) was added while stirring under ultrasonic irradiation to perform ultrasonic dispersing treatment for additional 30 min. The resulting dispersion was filtered and washed with ion-exchanged water. Then, excessive magnetic fluid was so removed to yield magnetic responsive particles [2].

2.2.5 Example 2-2

Substantially the same operation as in Example 1-2 was carried out, except that the magnetic responsive particles [2] prepared in Example 2-1 were used, to prepare anti-KL-6 antibody-sensitized magnetic responsive particles [2] and an anti-KL-6 antibody-sensitized magnetic responsive particle [2] dispersion.

2.2.6 Example 3-1

First, 2.0 g of Micropearl SP-203 (with a particle size of 3.02 $\mu$m and a CV of 4.9%, manufactured by SEKISUI CHEMICAL CO., LTD.) as resin particles was ultrasonically dispersed into 40.0 g of ion-exchanged water to prepare a core particle dispersion.
Subsequently, 5.0 mL of magnetic fluid EMG707 (manufactured by Ferrotec Corporation) was added while stirring under ultrasonic irradiation to perform ultrasonic dispersing treatment for additional 30 min. The resulting dispersion was filtered and washed with ion-exchanged water. Then, excessive magnetic fluid was so removed to yield magnetic responsive particles [3].

2.2.7 Example 3-2

Substantially the same operation as in Example 1-2 was carried out, except that the magnetic responsive particles [3] prepared in Example 3-1 were used, to prepare anti-KL-6 antibody-sensitized magnetic responsive particles [3] and an anti-KL-6 antibody-sensitized magnetic responsive particle [3] dispersion.

2.3.1 Comparative Example 1-1

As magnetic responsive particles, Magnosphere MS300 Tosyl (manufactured by JSR Life Sciences Corporation) was used as magnetic responsive particles [4].

2.3.2 Comparative Example 1-2

The above magnetic responsive particles [4] were ultrasonically dispersed at 3.0 wt% in PBS, and 0.5 mL thereof was transferred to a test tube. After the magnetic responsive particles [4] were collected using a magnet onto a wall surface of the test tube, the dispersion medium was removed. Then, 0.5 mL of a PBS solution containing an anti-KL-6 antibody (0.75 mg/mL) was added. The mixture was stirred overnight at 25°C for sensitization to prepare anti-KL-6 antibody-sensitized magnetic responsive particles [4]. After that, 1.5 mL of 1.0 wt% BSA solution was added, and the mixture was stirred at 25°C for 4 h. The sensitized magnetic responsive particles were collected using a magnet onto a wall surface of the test tube. Thereafter, the dispersion medium was removed, and 1.5 mL of 1.0 wt% BSA solution was newly added and dispersed. This operation was repeated three times to prepare an anti-KL-6 antibody-sensitized magnetic responsive particle [4] dispersion.

2.3.3 Comparative Example 2

First, 2.0 g of Micropearl SP-203 (with a particle size of 3.02 $\mu$m and a CV of 4.9%, manufactured by SEKISUI CHEMICAL CO., LTD.) as resin particles was ultrasonically dispersed into 40.0 g of 10 mM NaCl solution to prepare a core particle dispersion.
Subsequently, 8.0 mL of magnetic fluid EMG707 (manufactured by Ferrotec Corporation) was added while stirring under ultrasonic irradiation to perform ultrasonic dispersing treatment for additional 30 min. The resulting dispersion was allowed to stand for 3 min, and the supernatant was removed, and then, the particles were re-dispersed in ion-exchanged water, filtered, and washed with ion-exchanged water to obtain magnetic responsive particles [5].

2.3.4 Reference Example

Substantially the same operation as in Example 2-1 was carried out, except that the volume of magnetic fluid EMG707 (manufactured by Ferrotec Corporation) was changed to 2.0 mL, to produce magnetic responsive particles [6].
Table 1 shows the results of measuring the CV value for the volume-average particle size, the CV value for the weight-average particle size, the magnetic material content, the magnetic separability, and the dispersion rate of the particles obtained in Examples 1-1 to 3-2, Comparative Examples 1-1 to 2, or Reference Example.

[Table 1]

| Number | Type of particles | Average particle size (μm) | CV (%) for weight-average particle size | CV (%) for volume-average particle size | Magnetic material content (%) | Magnetic separability (%) | Dispersion rate (%) |
|---|---|---|---|---|---|---|---|
| Example 1-1 | Magnetic responsive particles [1] | 3.10 | 6.1 | 7.4 | 20.6 | 63 | 95 |
| Example 1-2 | Anti-KL-6 antibody-sensitized magnetic responsive particles [1] | 3.18 | 6.2 | 7.5 | - | 62 | 94 |
| Example 1-3 | Streptavidin-sensitized magnetic responsive particles [1] | 3.18 | 6.2 | 7.4 | - | 62 | 93 |
| Example 2-1 | Magnetic responsive particles [2] | 3.08 | 8.3 | 9.2 | 17.8 | 58 | 98 |
| Example 2-2 | Anti-KL-6 antibody-sensitized magnetic responsive particles [2] | 3.15 | 8.4 | 9.3 | - | 57 | 97 |
| Example 3-1 | Magnetic responsive particles [3] | 3.17 | 13.1 | 16.9 | 30.3 | 44 | 92 |
| Example 3-2 | Anti-KL-6 antibody-sensitized magnetic responsive particles [3] | 3.26 | 13.3 | 17.1 | - | 43 | 90 |
| Comparative Example 1-1 | Magnetic responsive particles [4] | 2.95 | 19.2 | 30.3 | 18.0 | 35 | 96 |
| Comparative Example 1-2 | Anti-KL-6 antibody-sensitized magnetic responsive particles [4] | 2.95 | 19.2 | 30.4 | - | 35 | 96 |
| Comparative Example 2 | Magnetic responsive particles [5] | 3.38 | 16.4 | 19.3 | 55.4 | 82 | 50 |

(continued)

| Number | Type of particles | Average particle size ($\mu$m) | CV (%) for weight-average particle size | CV (%) for volume-average particle size | Magnetic material content (%) | Magnetic separability (%) | Dispersion rate (%) |
|---|---|---|---|---|---|---|---|
| Reference Example | Magnetic responsive particles [6] | 3.04 | 5.8 | 6.3 | 9.8 | 40 | 98 |

Regardless of before or after the sensitization, the magnetic responsive particles in Examples 1 to 3 had a CV value for the weight-average particle size of 15% or less and a CV for the volume-average particle size of 20% or less, and had better magnetic separability than the magnetic responsive particles in Comparative Examples 1 having a comparable particle size but having a CV value for the weight-average particle size of larger than 15% and a CV value for the volume-average particle size of larger than 20%. It has thus been demonstrated that the magnetic responsive particles of the invention had a CV value for the weight-average particle size of 15% or less and had uniform magnetic material contained in each of the magnetic responsive particles, indicating excellent magnetic separability.

Meanwhile, the magnetic responsive particles in Comparative Example 2 were prepared such that the CV for the weight-average particle size was set to 15% or more by magnetic purification. Although high magnetic separability was exhibited due to increased magnetic material content, low dispersibility was elicited due to high particle specific gravity.

The magnetic responsive particles and the sensitized magnetic responsive particles in Example 3 had a CV value for the volume-average particle size of 15% or more and indeed had practical magnetic separability; however, their magnetic separability was less than those of the magnetic responsive particles and the sensitized magnetic responsive particles in Example 1 or 2. This suggests that as the volume-average particle size varies more, the magnetic separation becomes more ununiform.

The following procedures were used to evaluate practicality of some of the particles obtained in the above Examples or Comparative Examples were.

2.4.1 Reagent Evaluation

The following immunoassay was performed by using the sensitized magnetic responsive particles obtained in Example 1-2 or the sensitized magnetic responsive particles in Comparative Example 1-2, the particle surface of which had an anti-KL-6 antibody immobilized. The difference in luminescence level was determined between the case of immunoassay using a KL-6 concentration of 0 U/mL and the case of immunoassay using a standard solution at 5000 U/mL or each standard solution in which the antigen had been diluted to 10, 50, 100, 500, 1000, or 2500 U/mL.

Preparation of Ruthenium Complex-Labeled Anti-KL-6 Antibody

First, 0.5 mL of anti-KL-6 antibody-containing PBS solution (2.0 mg/mL) was added to a polypropylene tube. Next, 13 $\mu$L of Ru-NHS (1 mg/mL) was added. The mixture was stirred with shaking at 25°C, purified through a Sephadex G25 column, and then subjected to evaluation.

KL-6 Immunoassay:
The level of luminescence was measured with an automatic analyzer ("Picolumi III", manufactured by SEKISUI MEDICAL CO., LTD.), in which an electrochemiluminescence assay was used as a measurement principle. After 20 $\mu$L of a sample was added to 200 $\mu$L of reaction solution, 25 $\mu$L of anti-KL-6 antibody-conjugated magnetic particles were added. The mixture was reacted at 30°C for 9 min; 350 $\mu$L of 10 mM Tris buffer was added; and the particles were washed three times while trapped by a magnet. Next, 200 $\mu$L of ruthenium-labeled antibody solution containing 1.0 $\mu$g/mL ruthenium complex-labeled anti-KL-6 antibody was added. After 9-min reaction at 30°C, 350 $\mu$L of 10 mM Tris buffer was added. The particles were washed three times while trapped by a magnet. Then, 300 $\mu$L of 0.1 M tripropylamine-containing luminescence electrolytic solution was added, and the solution was fed onto a surface of electrode. Finally, the level of luminescence from the ruthenium complex bound to the particle was measured.

The following shows the results of practicality evaluation of the sensitized magnetic responsive particles in Example 1-2 or Comparative Example 1-2 evaluated in accordance with the above procedure.

[Table 2]

| KL-6 concentration [U/mL] | Example 1-2 | | Comparative Example 1-2 | |
|---|---|---|---|---|
| | Measured value | Difference in luminescence level from that at 0 U/mL | Measured value | Difference in luminescence level from that at 0 U/mL |
| 0 | 1097 | 0 | 1019 | 0 |
| 10 | 2364 | 1268 | 1026 | 7 |
| 50 | 8113 | 7016 | 1448 | 429 |
| 100 | 12954 | 11858 | 2301 | 1282 |
| 500 | 64891 | 63794 | 4281 | 3262 |
| 1000 | 144868 | 143771 | 9411 | 8392 |
| 2500 | 358228 | 357131 | 28978 | 27959 |
| 5000 | 621638 | 620541 | 90041 | 89022 |
| Luminescence level [counts] | | | | |

Compared with the particles in Comparative Example 1-2, the particles in Example 1-2 had a larger difference in luminescence level from that at 0 U/mL when reacted with the antigen at the same concentration, indicating higher sensitivity, namely superior reagent performance. The particles in Example 1-2 had a lower CV value for the weight-average particle size and a lower CV value for the volume-average particle size than the particles in Comparative Example 1-2, indicating excellent magnetic separability. The superior reagent performance has been elicited in the reagent evaluation probably because movement of particles in the liquid after sensitization by antibody is uniform and majority of the particles do not scatter and can be efficiently captured when the particles are trapped by a magnet.

Industrial Applicability

The invention can provide a highly sensitive immunoassay reagent that is easy to support a bio-related material and has increased separation/purification efficiency due to excellent magnetic separability by using magnetic responsive particles having a CV value for the weight-average particle size of 15% or less.

**Claims**

1. A sensitized magnetic responsive particle comprising:

   a magnetic responsive particle having a core particle and at least one magnetic layer disposed on the core particle, the magnetic layer comprising microparticles of a magnetic metal and/or an oxide thereof; and
   a substance that specifically interacts with an analyte, the substance being supported on the magnetic responsive particle,
   wherein a coefficient of variation in a weight-average particle size of the magnetic responsive particles is 15% or less.

2. The sensitized magnetic responsive particle according to claim 1, wherein the coefficient of variation in a volume-average particle size of the magnetic responsive particles is 20% or less.

3. A sensitized magnetic responsive particle comprising:

   a magnetic responsive particle having a core particle and at least one magnetic layer disposed on the core particle, the magnetic layer comprising microparticles of a magnetic metal and/or an oxide thereof; and
   a substance that specifically interacts with an analyte, the substance being supported on the magnetic responsive particle,
   wherein a coefficient of variation in a weight-average particle size of the sensitized magnetic responsive particles is 15% or less.

4. The sensitized magnetic responsive particle according to claim 3, wherein the coefficient of variation in a volume-average particle size of the sensitized magnetic responsive particles is 20% or less.

5. The sensitized magnetic responsive particle according to any one of claims 1 to 4, further comprising a nonmagnetic layer comprising a nonmagnetic metal oxide and/or an organic metal compound between the magnetic layer and the substance interacting specifically with the analyte.

6. The sensitized magnetic responsive particle according to any one of claims 1 to 5, wherein the substance interacting specifically with the analyte is chemically bonded onto the magnetic layer through a one-step or multi-step reaction.

7. The sensitized magnetic responsive particle according to any one of claims 1 to 6, wherein the substance interacting specifically with the analyte is bonded onto the nonmagnetic layer via one or multiple chemical bonds.

8. A heterogeneous immunoassay method using the sensitized magnetic responsive particle according to any one of claims 1 to 7.

9. An immunoassay reagent comprising the sensitized magnetic responsive particle according to any one of claims 1 to 7.

**INTERNATIONAL SEARCH REPORT**

| International application No. |
| --- |
| PCT/JP2020/013207 |

A.  CLASSIFICATION OF SUBJECT MATTER
G01N 33/543(2006.01)i; G01N 33/553(2006.01)i
FI: G01N33/553; G01N33/543 541A

According to International Patent Classification (IPC) or to both national classification and IPC

B.  FIELDS SEARCHED

Minimum documentation searched (classification system followed by classification symbols)
G01N33/543; G01N33/553

Documentation searched other than minimum documentation to the extent that such documents are included in the fields searched

| | |
| --- | --- |
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
| --- | --- | --- |
| X<br>Y | JP 2005-98974 A (SEKISUI CHEMICAL CO., LTD.)<br>14.04.2005 (2005-04-14) claims, paragraphs [0006]–<br>[0007], [0035], [0040], [0054] | 1, 3<br>2, 4-9 |
| Y | WO 2017/204209 A1 (JSR CORPORATION) 30.11.2017<br>(2017-11-30) paragraphs [0001]–[0009] | 2, 4-9 |
| Y | JP 2010-132513 A (SEKISUI CHEMICAL CO., LTD.)<br>17.06.2010 (2010-06-17) claims, paragraphs [0006]–<br>[0010], [0026] | 5-9 |
| Y | JP 2006-275600 A (JSR CORPORATION) 12.10.2006<br>(2006-10-12) paragraphs [0072], [0081], [0083] | 5-9 |

☒   Further documents are listed in the continuation of Box C.     ☒   See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 15 June 2020 (15.06.2020) | 23 June 2020 (23.06.2020) |

| Name and mailing address of the ISA/<br>Japan Patent Office<br>3-4-3, Kasumigaseki, Chiyoda-ku,<br>Tokyo 100-8915, Japan | Authorized officer |
| --- | --- |
| | Telephone No. |

Form PCT/ISA/210 (second sheet) (January 2015)

**EP 3 951 391 A1**

International application No.

PCT/JP2020/013207

C (Continuation).   DOCUMENTS CONSIDERED TO BE RELEVANT

| Category* | Citation of document, with indication, where appropriate, of the relevant passages | Relevant to claim No. |
|---|---|---|
| A | CN 101004414 A (EAST CHINA UNIVERSITY OF SCIENCE AND TECHNOLOGY) 25.07.2007 (2007-07-25) entire text, all drawings | 1-9 |

Form PCT/ISA/210 (continuation of second sheet) (January 2015)

**INTERNATIONAL SEARCH REPORT**
Information on patent family members

| International application No. |
| --- |
| PCT/JP2020/013207 |

| Patent Documents referred in the Report | Publication Date | Patent Family | Publication Date |
| --- | --- | --- | --- |
| JP 2005-93974 A | 14 Apr. 2005 | (Family: none) | |
| WO 2017/204209 A1 | 30 Nov. 2017 | EP 3467502 A1 paragraphs [0001]-[0011] CN 109154509 A | |
| JP 2010-132513 A | 17 Jun. 2010 | (Family: none) | |
| JP 2006-275600 A | 12 Oct. 2006 | (Family: none) | |
| CN 101004414 A | 25 Jul. 2007 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004205481 A

- JP 2013019889 A

**Non-patent literature cited in the description**

- Enzymatic Immunoassay. Igaku-Shoin Ltd, 1982